Europäisches Patentamt

European Patent Office

Office européen des brevets

(11) Numéro de publication: **0 267 819 B1**

# FASCICULE DE BREVET EUROPEEN

(45) Date de publication de fascicule du brevet:
**15.05.91**

(51) Int. Cl.5: **C07C 7/11**, C07C 9/04, B01D 53/14

(21) Numéro de dépôt: **87402207.2**

(22) Date de dépôt: **05.10.87**

(54) **Procédé integré de traitement d'un gaz humide renfermant du méthane dans le but d'en éliminer l'eau.**

(30) Priorité: **16.10.86. FR 8614504**

(43) Date de publication de la demande:
**18.05.88 Bulletin 88/20**

(45) Mention de la délivrance du brevet:
**15.05.91 Bulletin 91/20**

(84) Etats contractants désignés:
**DE GB IT NL**

(56) Documents cités:
**EP-A- 0 053 424**
**FR-A- 2 550 956**
**US-H- 421 383**

(73) Titulaire: **INSTITUT FRANCAIS DU PETROLE**
**4, Avenue de Bois-Préau**
**F-92502 Rueil-Malmaison(FR)**

(72) Inventeur: **Rojey, Alexandre**
**29-33 rue Henri Regnault**
**F-92380 Garches(FR)**
Inventeur: **Larue, Joseph**
**97, Grande Rue**
**F-78240 Chambourcy(FR)**

## Description

Le traitement d'un gaz, que ce soit du gaz naturel ou du gaz de raffinerie, comprend généralement une étape de déshydratation. Cette déshydratation a pour but d'éviter les problèmes de formation d'hydrates et de corrosion en cours de transport.

Le traitement peut également comprendre une étape de séparation d'hydrocarbures supérieurs, par exemple de $C_2$ à $C_5$ (GPL), par réfrigération. Ce traitement a pour but soit d'ajuster le point de rosée hydrocarbures pour éviter une condensation au cours du transport, soit de récupérer une fraction GPL plus valorisable que le gaz traité.

Il peut être également nécessaire d'ajuster la teneur en gaz acides, particulièrement $H_2S$ et $CO_2$.

Ces étapes de traitement sont généralement indépendantes. Selon des techniques connues :
- La déshydratation peut être réalisée par un lavage à l'aide d'un solvant qui est le plus souvent un glycol tel que le diéthylène glycol (DEG). Ce solvant doit être régénéré par distillation.
- La séparation des GPL est réalisée par réfrigération à une température qui doit être d'autant plus basse que le taux de récupération souhaité de ces GPL est élevé.
- La désacidification est réalisée par un lavage à l'aide d'un solbant physique (par exemple éther diméthylique de polyéthylène glycol = DMEPEG ou carbonate de propylène) ou chimique (amine). Ce solvant doit être régénéré par distillation.

Selon la demande de brevet français 2 550 956, la purification d'un gaz naturel peut être mise en oeuvre par lavage du gaz avec du méthanol liquide. Le méthanol absorbe les impuretés et il est ensuite nécessaire de le régénérer par distillation.

Selon la demande de brevet publiée des Etats-Unis d'Amérique B 421 383, un gaz humide est lavé avec du méthanol pour débarrasser le gaz d'au moins une partie de son eau. On recueille une phase eau-méthanol qu'il faut régénérer par distillation. La régénération du solvant peut se faire, par exemple, par la méthode de la demande de brevet européen 0 053 424.

Il a été découvert que la déshydratation, seule ou associée à la séparation des GPL et/ou à la désacidification, peut être réalisée en une seule opération de traitement, ce qui permet un gain important en investissement ainsi qu'en encombrement et poids des installations, ce qui peut être particulièrement important dans un contexte tel que celui de la production de gaz naturel en mer.

Il a été également découvert que ces opérations de traitement peuvent être réalisées avec un taux de solvant qui est très inférieur au taux de solvant généralement pratiqué dans les opérations de lavage par solvant.

Il a été également découvert que le solvant utilisé peut être régénéré sans avoir recours à une étape de distillation ce qui présente également de nombreux avantages : gain en investissement ainsi qu'en encombrement et poids des installations, réduction de la consommation d'énergie, possibilité d'éviter un chauffage par flamme, ce qui peut également représenter un avantage important dans un contexte tel que celui de la production de gaz naturel en mer.

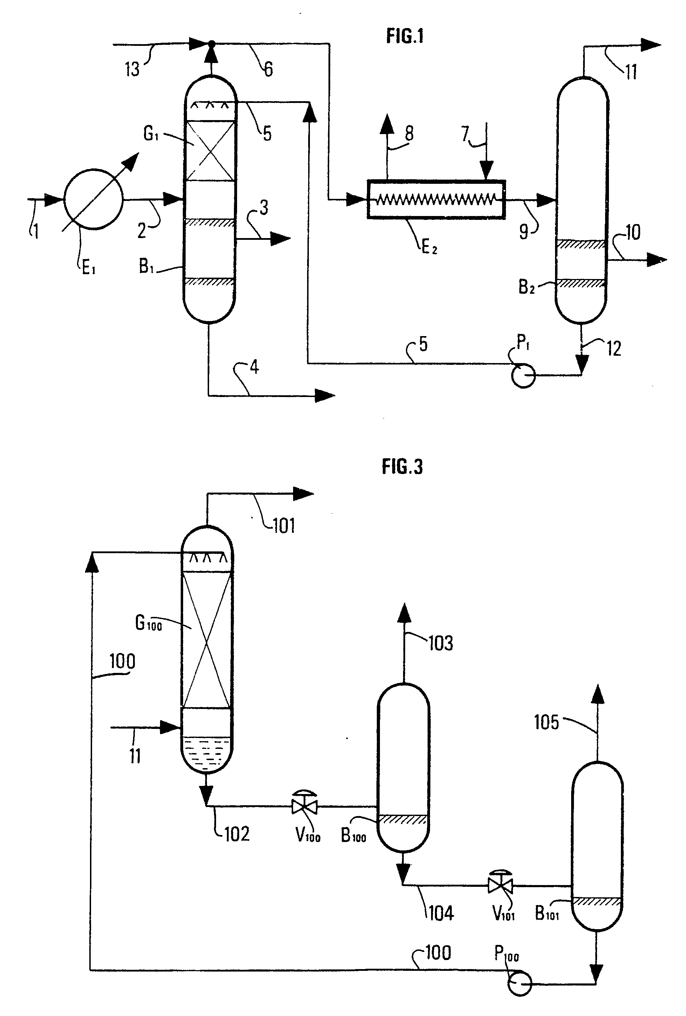

Les Figures 1 à 5 illustrent divers modes de mise en oeuvre de l'invention.

Le principe du procédé selon l'invention est illustré par le schéma de la Figure 1, appliqué à titre d'exemple à un gaz naturel renfermant des hydrocarbures supérieurs associés.

Le gaz à traiter arrive par le conduit 1. Il est refroidi par le fluide de refroidissement disponible, par exemple eau ou air, dans l'échangeur E1 d'où il ressort par le conduit 2. Cette première étape de refroidissement permet de séparer une fraction gasoline liquide formée par les hydrocarbures condensables dans les conditions de température et de pression établies à la sortie de l'échangeur E1, fraction qui est évacuée par le conduit 3 et une fraction aqueuse qui décante au fond du bac B1 et qui est évacuée par le conduit 4. Le gaz restant est saturé en eau.

Cette première étape de refroidissement n'est pas toujours obligatoire, en particulier si le gaz à traiter est disponible à une température déjà relativement basse.

Le gaz est ensuite contacté avec une phase liquide formée par le solvant contenant de l'eau provenant du bac B2 et arrivant par le conduit 5, dans une zone de contact G1 formée par exemple par un garnissage. Le gaz sortant de la zone de contact renferme de la vapeur d'eau et de solvant, le plus souvent en quantités voisines de la saturation. Le contact entre le gaz et la phase liquide dans la zone de contact G1 est effectué de préférence à contre-courant.

L'ensemble des opérations effectuées dans la zone de contact G1 et dans la zone de décantation B1 n'est pas nécessairement réalisé dans une enceinte unique. En particulier les phases liquides recueillies à la sortie de l'échangeur E1 peuvent être séparées dans une enceinte distincte. D'autre part différentes géométries permettant de favoriser le décantation des phases liquides par exemple en augmentant le temps de séjour, peuvent être adoptées pour la zone de décantation B1.

Il a été découvert que le solvant entraîné en phase gazeuse à la sortie de la zone de contact G1 peut suffire à éviter les problèmes de formation d'hydrates liés à l'étape de refroidissement destinée à séparer la fraction GPL.

Il a été également découvert que ladite étape de refroidissement permet, à condition d'éviter les problèmes de formation d'hydrates par les moyens spécifiés, d'assurer également la déshydratation du gaz traité.

Le gaz sortant de la zone de contact G1, saturé en eau et en solvant est évacué par le conduit 6.

Il est alors refroidi dans l'échangeur E2 par un fluide frigorigène qui arrive par le conduit 7 et repart par le conduit 8.

Il ressort de l'échangeur E2 par le conduit 9. Le refroidissement opéré dans l'échangeur E2 permet de condenser une franction GPL qui est évacuée par le conduit 10. Il permet également de condenser la majeure partie de l'eau et du solvant qui étaient contenus dans le gaz arrivant dans l'échangeur E2 par le conduit 6 ; on obtient ainsi une phase liquide formée par un mélange d'eau et de solvant non miscible avec la phase liquide de GPL et qui est évacuée du bac B2 par le conduit 12 en étant reprise par la pompe P1 et recyclée par le conduit 5 vers la zone de contact G1.

Dans la zone de contact G1, la phase liquide recyclée arrivant par le conduit 5 et formée par un mélange de solvant et d'eau est contactée avec le gaz entrant par le conduit 2, qui est saturé en eau mais qui ne contient pas de solvant. Le solvant de la phase liquide recyclée se trouve au moins en majeurs partie vaporisé et entraîné par la phase gazeuse tandis que l'eau de la phase liquide recyclée qui ne peut être entraînée par la phase gazeuse déjà saturée en eau est recueillie au fond du bac B1, au moins en majeure partie débarrassée de solvant. La concentration en solvant de l'eau sortant par le conduit 4 est couramment inférieure à 10 % et de préférence inférieure à 5 %, par exemple 0,01 à 5 %. La séparation entre l'eau et le solvant est ainsi réalisée par contact avec le gaz à traiter sans avoir à régénérer ledit solvant par distillation.

Dans le procédé selon l'invention, le solvant subit un cycle : il est successivement vaporisé par le gaz traité dans le contacteur G1, condensé en même temps que l'eau au cours du refroidissement dans l'échangeur E2 pour former une phase liquide séparée de la phase gazeuse et de la phase hydrocarbure dans le ballon B2, et recyclée vers le contacteur par la pompe P1 dans le conduit 5. Bien que le solvant soit ainsi recyclé dans le procédé, un appoint dudit solvant peut être apporté par le conduit 13, pour compenser les pertes provenant des traces de solvant entraînées dans les conduits 4, 10 et 11 : la quantité d'appoint apportée par le conduit 13 dépend des conditions de fonctionnement du procédé : elle est généralement inférieure à 20 % de la quantité de solvant contenue dans la solution recyclée circulant dans le conduite 5, et de préférence inférieure à 5 %. La solution de solvant recyclée par le conduit 5 est formée par la majorité de l'eau et du solvant présents dans le gaz à la sortie du contacteur dans le conduit 6 et que est condensée par le refroidissement dans l'échangeur E2 ; en conséquence, les quantités d'eau et de solvant contenues dans la solution recyclée circulant dans le conduit 5 sont pratiquement les mêmes que celles contenues dans le gaz sous forme de vapeur à la sortie du contacteur.

La quantité de solvant contenue dans la phase liquide recyclée circulant dans le conduit 5 correspond à la concentration par rapport à l'eau nécessaire à l'inhibition de la formation d'hydrates et/ou de glace dans le gaz à traiter pendant son refroidissement dans l'échangeur E2 ; cette concentration doit être d'autant plus importante que la température du ballon B2 est plus basse ; elle est de préférence comprise entre 10 et 90 % en poids. Elle peut être évaluée par différentes méthodes connues de l'homme de l'art, par exemple par la formule d'Hammerschmidt couramment utilisée dans le domaine du gaz naturel. La quantité de solvant contenu dans ladite phase liquide recyclée est donc liée à la quantité d'eau contenue dans le gaz à traiter sous forme de vapeur et entrant dans le contacteur et elle est du même ordre de grandeur. Le procédé selon l'invention ne réalise donc pas un lavage du gaz par une phase solvant ayant pour but d'absorber l'eau contenue dans le gaz, ce qui conduit à mettre en oeuvre des quantités de solvant très supérieures à la quantité d'eau contenue dans le gaz, couramment 10 à 100 fois plus. Dans le procédé selon l'invention, la quantité de solvant recyclée est telle que le gaz à traiter sortant du contacteur G1 par le conduit 6 contient, sous forme vapeur, la majeure partie du solvant recyclé (couramment plus de 90 %) et la majeure partie de l'eau qu'il contenait à l'entrée du contacteur G1 (couramment plus de 50 %).

En définitive, le procédé selon l'invention comprend les étapes suivantes :

a) On contacte le gaz à traiter, de préférence à contre-courant, avec une phase liquide recyclée renfermant à la fois de l'eau et un solvant, les conditions de contact étant choisies de manière à vaporiser et transférer au moins 90 % dudit solvant dans ledit gaz, on évacue la phase liquide aqueuse résultant dudit contact et renfermant moins de 10% de la quantité recyclée dudit solvant, et on recueille ledit gaz renfermant à l'état vaporisé au moins 90 % dudit solvant contacté avec le gaz, ledit solvant étant un composé non-hydrocarbure autre que l'eau, miscible à l'eau. De préférence, ledit contact est caractérisé en ce que les quantités d'eau et de solvant contenues dans ladite phase liquide recyclée sont pratiquement les mêmes que celles contenues dans ledit gaz recueilli après contact.

b) On refroidit le gaz provenant de l'étape (a) en produisant la condensation d'une fraction liquide formée essentiellement de solvant et d'eau.

c) On sépare le gaz de ladite fraction liquide et on recycle à l'étape (a) ladite fraction liquide renfermant à la fois du solvant et de l'eau.

De préférence ladite fraction liquide consiste essentiellement en solvant et eau.

De préférence on ajoute du solvant pour compenser les pertes. Avantageusement cette addition se fait sur le gaz venant de l'étape (a) avant de procéder à l'étape (b).

Pour ajuster le taux de solvant vaporisé lors du contact de l'étape (a), on peut agir notamment sur la température, la pression et les proportions relatives de gaz et de phase liquide recyclée.

Lorsque le gaz renferme, en outre, au moins un hydrocarbure autre que le méthane, condensable dans les conditions de l'étape (b) en formant une fraction liquide d'hydrocarbure distincte de ladite fraction formée essentiellement de solvant et d'eau, on sépare ladite fraction liquide d'hydrocarbure et on la décharge du procédé.

Le principe du procédé selon l'invention est illustré par l'exemple 1.

EXEMPLE

Dans cet exemple, on procède selon le schéma représenté sur la Figure 1. Le gaz à traiter, qui est un gaz associé d'un puits de pétrole, arrive par le conduit 1 ; sa température est de 60 $^\circ$C, sa pression est de 4 MPa, son débit est de 56 100 kg/h ; il est saturé en eau. Il est refroidi par échange thermique avec de l'eau jusqu'à une température de 20 $^\circ$C dans l'échangeur de chaleur E1 d'où il ressort par le conduit 2. Ce refroidissement provoque la condensation de 2000 kg/h de gasline liquide d'une part, et d'eau liquide d'autre part (207,9 kg/h) : le mélange formé du gaz non condensé et des deux phases liquides entre dans le bac B1 : les deux phases liquides sont séparées dans le fond du bac B1, la phase de gasoline est évacuée par le conduit 3. Le gaz restant, qui est saturé en eau (28,5 kg/h), est contacté à contre-courant à 20 $^\circ$C dans un garnissage G1 avec une phase liquide formée par un mélange de méthanol et d'eau provenant du bac B2 et arrivant par le conduit 5 ; la phase aqueuse qui décante au fond du ballon B1 sort par le conduit 4, son débit est de 237 kg/h dont 236,3 kg/h d'eau et 0,7 kg/h de méthanol : elle est constituée en partie (207,9 kg/h) de l'eau condensée arrivant avec le gaz par le conduit 2 et en partie (29,1 kg/h) d'eau provenant du contact gaz-liquide dans le garnissage G1 (renfermant lesdits 0,7 kg/h de méthanol) ; le mélange eau-méthanol circulant dans le conduit 5 contient 28 % en poids d'eau et 72 % en poids de méthanol, son débit est de 88,2 kg/h. Le gaz (53.951,2 kg/h) sort du bac B1 par le conduit 6 saturé en eau (24,8 kg/h) et en méthanol (62,8 kg/h soit 98,9 % du méthanol chargé) ; il est refroidi jusqu'à une température de -40 $^\circ$C dans un échangeur de chaleur E2 par un fluide réfrigérant extérieur qui arrive par le conduit 7 et ressort par le conduit 8. Ce refroidissement provoque une condensation d'hydrocarbure liquide (liquide de gaz naturel) et de la majeure partie de l'eau et du méthanol.

Le gaz purifié et les deux fractions liquides sont séparés dans le bac B2 ; la fraction eau-méthanol sort du bac B2 par le conduit 12, est reprise par la pompe P1 et recyclée par le conduit 5 vers le bac B1 ; la fraction hydrocarbure liquide est évacuée par le conduit 10, son débit est de 15 600 kg/h.

Un appoint de solvant destiné à compenser les pertes et à maintenir des conditions stables de fonctionnement est effectué par la ligne 13. Comme le montre cet exemple, le procédé permet d'opérer avec une circulation de solvant réduite puisque le rapport molaire du débit de solvant qui est véhiculé par la pompe P1 au débit de gaz est égal à 1/780 environ.

De manière générale ce rapport reste inférieur à 1/10 et peut même être inférieur à 1/100, comme le montre l'exemple précédent, il est généralement compris entre 1/5000 et 1/10, par exemple entre 1/1000 et 1/10.

Un autre avantage essentiel du procédé tel qu'il ressort de cet exemple est que la phase solvant est régénérée sans qu'il soit nécessaire de recourir à une distillation.

La quantité de solution de solvant recyclée circulant dans le conduit 5 étant faible en comparaison de la quantité de gaz, il n'est pas nécessaire que la totalité du gaz à traiter soit mise en contact avec la solution recyclée dans la zone de contact G1, et une partie du gaz peut être court-circuitée. Le principe de cette configuration est illustré par la Figure 5 : le gaz à traiter arrivant par le conduit 1 est séparé en deux ; une partie entre dans l'échangeur E1, puis dans le contacteur par le conduit 2 et est mise en contact avec la solution recyclée circulant dans le conduit 5 exactement comme dans le cas de la Figure 1 ; l'autre partie du gaz à traiter passe dans le conduit 14 et est refroidie dans l'échangeur E3, ce qui permet de condenser une fraction d'eau qui est séparée dans le bac B3 et évacuée par le conduit 16. Le gaz sortant par le conduit 15, saturé en eau, est mélangé au gaz provenant du contacteur G1 et qui contient le solvant en phase gazeuse ; l'ensemble du flux de gaz, circulant dans le conduit 6, entre dans l'échangeur E2 pour refroidissement comme dans le cas de la Figure 1. La fraction du gaz traité mise en contact avec la solution liquide recyclée peut représenter dans ce cas une fraction mineure du gaz à traiter et être comprise par exemple entre 10 et 50%.

Cette configuration comporte plusieurs avantages : elle permet de régénérer le solvant avec la quantité strictement nécessaire de gaz ; de plus, elle conduit à un rapport liquide/gaz plus proche de celui utilisé avec les contacteurs classiques tels que ceux à garnissage par exemple ; d'autre part les conditions de température du contacteur peuvent être ajustées à une valeur différente de celle du ballon B3 pour optimiser l'ensemble du schéma.

Suivant la composition du gaz traité, la quantité de GPL condensée dans le ballon B2 peut être beaucoup plus importante que la quantité de solution de solvant et d'eau également condensée, couramment de 10 à 200 fois plus. Bien que le solvant soit peu soluble dans la phase liquide de GPL, la quantité de solvant qui y est dissoute peut représenter couramment de 1/50 à 1/2 fois la quantité totale de solvant condensée dans le ballon B2. Le solvant content dans la phase liquide de GPL circulant dans le conduit 10 peut être récupéré par lavage de la phase liquide de GPL par de l'eau ce qui produit d'une part une phase liquide de GPL débarrassée de solvant et d'autre part une solution d'eau et de solvant qui peut être mélangée à la solution recyclée circulant dans le conduit 5 et régénérée dans le contacteur G1, en évitant ainsi à nouveau le recours à la régénération par distillation.

Dans le schéma de la Figure 1, le froid nécessaire a été supposé être apporté dans l'échangeur E2 par un fluide frigorigène qui arrive par le conduit 7 et repart par le conduit 8. D'autres sources de froid peuvent être également mises en oeuvre dans le procédé selon l'invention, et en particulier si elles sont produites par le gaz à traiter lui-même, telles que par exemple une turbine de détente, ou une vanne de détente. Un exemple de configuration avec turbine de détente est illustré par la Figure 5 : par rapport à la Figure 1, la réfrigération du gaz est assurée par détente du gaz dans une turbine ; le gaz sortant du contacteur G1 est refroidi dans un échangeur E4 par échange avec le gaz traité, détendu dans la turbine T1 avec production d'énergie mécanique, et entre dans le ballon B2 par le conduit 9. Le gaz traité sort du ballon B2 par le conduit 11 et se réchauffe dans l'échangeur E4 dont il ressort par le conduit 17. La configuration avec vanne de détente est similaire à la configuration avec turbine de détente, mais la turbine est remplacée par une vanne de détente qui produit une détente isenthalpique du gaz.

Différents solvants autres que le méthanol peuvent être utilisés.

Le solvant doit présenter une grande miscibilité avec l'eau et peut être par exemple un alcool autre que le méthanol, tel que l'éthanol, le propanol, le butanl, une cétone telle que l'acétone ou la méthyléthylcétone, ou encore d'autres solvants polaires tels que la N-méthylpyrrolidone, la diméthylformamide ou la morpholine.

Le solvant peut être également consitué par un mélange de solvants.

Il est avant tout nécessaire que le solvant soit au moins partiellement miscible (de préférence totalement miscible) avec l'eau et soit de préférence peu miscible avec les hydrocarbures de manière à former au cours de l'étape (b) deux phases liquides lorsque la concentration en hydrocarbures autres que le méthane est relativement élevée.

Toutefois dans le cas d'un gaz contenant relativement peu d'hydrocarbures autres que le méthane, le procédé peut s'appliquer sans qu'il y ait production d'une phase hydrocarbure liquide. Il n'est pas indispensable dans ce cas que le solvant soit peu miscible avec les hydrocarbures.

L'utilisation d'un solvant moins volatil que le méthanol présente l'avantage de limiter la quantité de solvant entraînée avec le gaz traité, pour une température de réfrigération donnée. Ceci est avantageux même si le solvant est récupéré au terminal de réception car cela permet de réduire la fréquence des réapprovisionnements en solvant sur le site de production. Moins la température finale de réfrigération est basse, plus il est avantageux d'utiliser un solvant lourd.

Toutefois lorsque le solvant est moins volatil que l'eau, la phase vapeur sortant de la zone de contact G1 au cours de l'étape de régénération est habituellement moins riche que la phase liquide recyclée par la pompe P1. Il en résulte une régénération incomplète et l'eau évacuée contient encore du solvant.

Il a été découvert et c'est là un autre objet de la présente invention que, notamment dans le cas précité, il est avantageux d'utiliser un solvant formant un azéotrope avec l'eau tel que l'éthanol, le 2-méthoxyéthanol, le propanol, le butanol, l'alcool propargylique, la pyridine, la pipéridine.

Dans ces conditions en effet lorsque l'on recycle à la zone de régénération une phase liquide avec une composition en eau et en solvant proche de la composition de l'azéotrope, il est possible au cours de l'étape de régénération d'entraîner en phase vapeur à la sortie de la zone de contact G1 un mélange d'eau et de solvant sensiblement de même composition que celle de la phase liquide recyclée.

Lorsque le solvant est choisi de manière à former avec l'eau un azéotrope comprenant relativement peu de solvant de manière à réduire les pertes en solvant, la quantité de solvant entraînée en phase gazeuse au cours de l'étape de régénération devient relativement faible et la concentration en solvant de la solution obtenue à l'issue de l'étape de réfrigération peut être insuffisante pour prévenir la formation des hydrates à la température de réfrigération la plus basse.

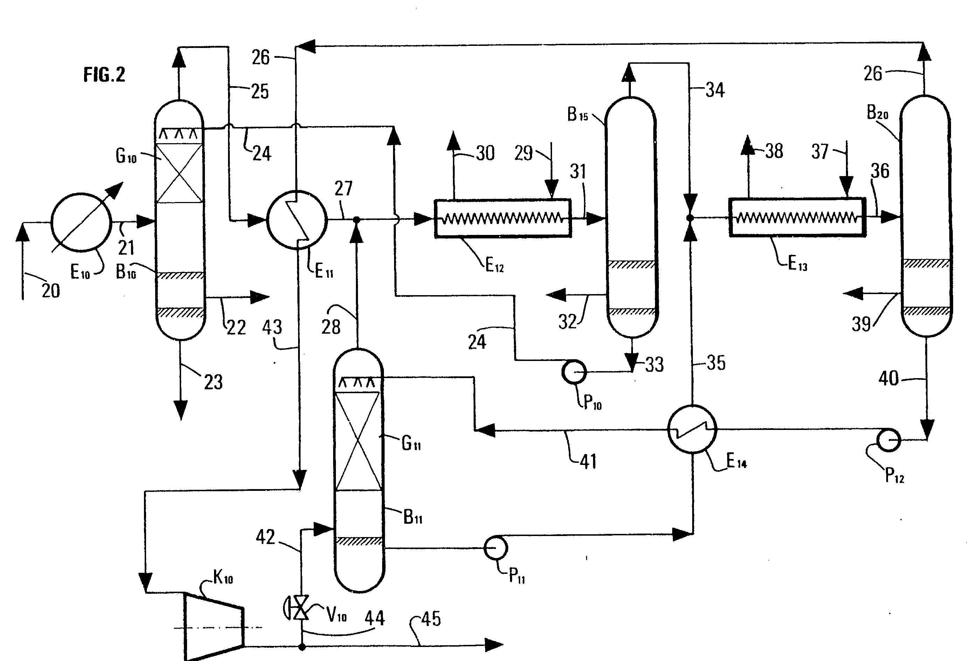

On peut dans ce cas avoir recours à la version du procédé illustrée par le schéma de la Figure 2, appliquée ici à un gaz renfermant du méthane et des hydrocarbures condensables.

Le gaz à traiter arrive par le conduit 20. Il est refroidi par le fluide de refroidissement disponible (eau ou air) dans l'échangeur E10 d'où il ressort par le conduit 21, qui débouche sur le bac B10. Cette première étape de refroidissement permet de séparer une fraction gazoline liquide formée par les hydrocarbures condensables dans les conditions de température et de pression établies à la sortie de l'échangeur E10, qui est évacuée par le conduit 22, et une fraction aqueuse qui décante au fond du bac B10 et qui est évacuée par le conduit 23. Le gaz restant est saturé en eau.

Le gaz est ensuite contacté avec une phase liquide formée par le solvant contenant de l'eau provenant du bac B15 et arrivant par le conduit 24 dans une zone de contact G10 formée par exemple par un garnissage. Le gaz sortant de la zone de contact est saturé en eau et en solvant.

Le gaz sortant de la zone de contact G10 saturé en eau et en solvant est évacué par le conduit 25.

Il est alors refroidi dans l'échangeur E11 par échange de chaleur avec le gaz traité provenant du bac B20 et arrivant par le conduit 26. Il ressort de l'échangeur E11 par le conduit 27 et est mélangé avec une fraction gazeuse provenant du bac B11 et arrivant par le conduit 28.

Le mélange gazeux ainsi obtenu est envoyé dans l'échangeur E12 dans lequel il est refroidi par un fluide frigorigène qui arrive par le conduit 29 et repart par le conduit 30. Il ressort de l'échangeur E12 par le conduit 31.

Le refroidissement opéré dans l'échangeur E12 permet de condenser une fraction GPL qui est évacuée par le conduit 32. Il permet également de condenser la majeure partie de l'eau et du solvant. On obtient ainsi une phase liquide formée par un mélange d'eau et de solvant qui décante dans le bac B15 et qui est évacuée du bac B15 par le conduit 33 en étant reprise par la pompe P10 et recyclée par le conduit 24 vers la zone de contact G10.

Dans la zone de contact G10, la phase formée par un mélange de solvant et d'eau est contactée à contre-courant avec un gaz saturé en eau mais ne contenant pas de solvant à l'entrée. Le débit de gaz étant très supérieur au débit de ladite phase liquide, le solvant se trouve vaporisé et entraîné par la phase gazeuse tandis que l'eau est recueillie au fond du bac B10. La séparation entre l'eau et le solvant est ainsi réalisée par contact avec le gaz à traiter.

Le gaz provenant du bac B15 est évacué par le conduit 34 et subit une étape de traitement supplémentaire.

Il est tout d'abord mélangé avec une fraction solvant en phase liquide provenant du bac B11 et arrivant par le conduit 35.

Le mélange gaz-liquide ainsi obtenu subit un refroidissement complémentaire dans l'échangeur E13 dans lequel il est refroidi par un fluide frigorigène qui arrive par le conduit 37 et repart par le conduit 38.

Il ressort de l'échangeur E13 par le conduit 36 et débouche dans le bac de décantation B20.

Le refroidissement opéré dans l'échangeur E13 permet de condenser une fraction GPL complémentaire qui est évacuée par le conduit 39.

Il permet également de condenser une fraction complémentaire d'eau et de solvant. Le mélange d'eau et de solvant ainsi obtenu est plus concentré en solvant que celui qui à été recueilli par le conduit 33 à l'issue de l'étape de réfrigération opérée dans l'échangeur E12, du fait de l'introduction de solvant liquide par le conduit 35. Ceci permet d'obtenir à la sortie de l'échangeur E13 une température plus basse que celle qui est obtenue à la sortie de l'échangeur E12 sans risque de formation d'hydrates. Le mélange d'eau et de solvant obtenu à la sortie de l'échangeur E13 décante dans le bac B20 et est évacué par le conduit 40 en étant repris par la pompe P12 ; il est envoyé à l'échangeur E14 d'où il ressort par le conduit 41 et est envoyé à la zone de contact G11. La zone de contact à contre-courant G11 est formée par exemple par un garnissage.

Dans la zone de contact G11 le mélange de solvant et d'eau arrivant par le conduit 41 est mis en contact avec une fraction gazeuse arrivant par le conduit 42.

Cette fraction gazeuse est prélevée sur le gaz traité provenant par la conduite 26 du bac B20 et, qui, à la sortie de l'échangeur E11, est évacué par le conduit 43 et est comprimé avant expédition dans l'étape de compression K10.

A la sortie de l'étape de compression K10, une fraction du gaz traité est prélevée par le conduit 44, détendue à travers la vanne de détente V10 et envoyée à la zone de contact G11. La majeure partie du gaz traité est évacuée par le conduit 45.

La fraction gazeuse du conduit 45 est peu chargée en eau et en solvant. Le solvant utilisé dans cette version du procédé étant plus lourd que l'eau, le contact à contre-courant entre le mélange d'eau et de solvant arrivant par le conduit 41 et la fraction gazeuse arrivant par le conduit 42 permet de stripper l'eau mélangée au solvant, eau qui se retrouve dans la fraction gazeuse évacuée par le conduit 28, le solvant non vaporisé étant recueilli au fond du bac B11.

Ce solvant est évacué par la pompe P11, passe dans l'échangeur E14 et est envoyé par le conduit 35 pour être mélangé avec le gaz arrivant par le conduit 34. Il est ainsi possible comme cela a été indiqué d'obtenir à la sortie de l'échangeur E13 un mélange en phase liquide d'eau et de solvant plus concentré en solvant que le mélange d'eau et de solvant recueilli à la sortie de l'échangeur E12.

La quantité d'eau contenue dans le gaz évacué du bac B15 par la conduite 34 étant faible, la quantité de solvant qu'il est nécessaire d'envoyer par le conduit 35 est également faible ainsi que la quantité de gaz nécessaire pour stripper le mélange en phase liquide d'eau et de solvant recueilli à la sortie de l'échangeur E13.

En définitive, cette version du procédé est caractérisée en ce qu'elle comprend les étapes (a) à (c) de la version de base du procédé. Toutefois avant le refroidissement de l'étape (b), on ajoute une fraction gazeuse provenant de l'étape (g) ci-après. En outre cette version du procédé comprend les étapes (d) à (g) complémentaires telles que :

d) On mélange la fraction de gaz provenant de l'étape de séparation (c) avec une fraction contenant du solvant en phase liquide provenant de l'étape (g).

e) On refroidit le mélange liquide-gaz provenant de l'éape (d) en produisant une fraction liquide formée essentiellement de solvant et d'eau et une fraction gazeuse.

f) Après l'étape de réfrigération (e), on sépare les fractions obtenues.

g) On évacue une partie de la fraction gazeuse séparée à l'étape (f), comme gaz épuré, et on met en contact une autre partie de ladite fraction gazeuse séparée à l'étape (f) avec au moins une partie de la fraction liquide de solvant et d'eau, séparée à l'étape (f), à température plus élévée que celle de l'étape (f) en produisant une fraction liquide formée essentiellement de solvant et une fraction gazeuse, et on recycle ladite fraction liquide à l'étape (d) et ladite fraction gazeuse à l'étape (b).

Cette version du procédé selon l'invention est illustrée par l'exemple 2.

EXEMPLE 2

Dans cet exemple, on procède selon le schéma représenté sur la Figure 2. Cependant, le gaz traité contenant peu d'hydrocarbures autres que le méthane (gaz sec), il n'y a pas de condensation d'hydrocarbures liquides dans les bacs B10, B15 et B20. Le gaz à traiter arrive par le conduit 20 : sa température est de 90 °C, sa pression est de 5 MPa, son débit est de 126 000 kg/h. Le gaz est saturé en eau, son point de rosée hydrocarbure à la pression de 5 MPa est inférieur à -10 °C (gaz sec). Le gaz est refroidi par échange thermique avec de l'eau de refroidissement dans l'échangeur de chaleur E10 d'où il ressort par le conduit 21 à une température de 50 °C. Ce refroidissement provoque la condensation d'une fraction d'eau liquide. Le gaz et la phase aqueuse sont séparés dans le bac B10. Le gaz restant, saturé en eau, est contacté à contre-courant dans le garnissag G10 avec une phase liquide formée par le solvant contenant de l'eau provenant du bac B15 et arrivant par le conduit 24 : le solvant utilisé dans ce cas est le 2-méthoxyéthanol dont la formule brute est $C_3H_8O_2$. La solution eau - 2-méthoxyéthanol circulant dans le conduit 24 contient 22,2 % en poids de 2-méthoxyéthanol, sont débit est de 340 kg/h. La phase aqueuse qui est recueillie au fond du ballon B10, sort par le conduit 23, son débit est de 1560 kg/h ; elle est constituée d'une part de la fraction d'eau condensée arrivant avec le gaz par le conduit 21 et d'autre part par l'eau provenant du contact gaz-liquide dans le garnissage G10. Le gaz sortant du bac B10 par le conduit 25, saturé en eau et en 2-méthoxyéthanol, est refroidi dans un premier échangeur de chaleur E11 par échange thermique avec le gaz traité provenant du bac B20, et arrivant par le conduit 26. Il ressort de l'échangeur par le conduit 27 et est mélangé avec une fraction gazeuse provenant du bac B11 et arrivant par le conduit 28.

Le mélange gazeux ainsi obtenu est refroidi jusqu'à une température de 15 °C dans l'échangeur E12 par échange thermique avec un fluide réfrigérant extérieur entrant par le conduit 29 et ressortant par le conduit 30 ; il ressort de l'échangeur par le conduit 31 ; les refroidissments successifs dans les échangeurs E11 et E12 conduisent à une condensation de mélange eau - 2-méthoxyéthanol : cette phase liquide est séparée du gaz dans le bac B15 et est évacuée par le conduit 33 ; elle est reprise par la pompe P10 et recyclée par le conduit 24 vers le garnissage du bac B10. Le gaz, partiellement déshydraté, sort du bac B15 par le conduit 34, et est mis en contact avec un mélange eau - 2-méthoxyéthanol en phase liquide provenant du bac B11 et arrivant par le conduit 35 : le débit de ce mélange est de 52 kg/h, sa composition est de 60 % en poids de 2-méthxyéthanol et 40 % en poids d'eau. Le mélange gaz-liquide ainsi obtenu est refroidi jusqu'à une température de 0 °C dans l'échangeur de chaleur E13 par échange thermique avec un fluide réfrigérant extérieur entrant par le conduit 37 et ressortant par le conduit 38 ; il en ressort par la conduit 36 et entre le bac B20. Le refroidissement du mélange gaz-liquide dans l'échangeur E13 permet de condenser une fraction complémentaire d'eau et de 2-méthoxyéthanol. La fraction liquide est séparée dans le bac B20 et en ressort par le conduit 40 : ce liquide contient 46 % en poids de 2-méthoxyéthanol. Cette fraction liquide est reprise par la pompe P12, est envoyée dans l'échangeur E14 dans lequel elle se réchauffe par échange thermique avec la solution circulant dans le conduit 35, et est envoyée par le conduit 41 à la zone de contact G11, formée par un garnissge où elle est mise en contact avec une fraction gazeuse provenant du conduit 42, et dont le débit est de 4500 kg/h.

Dans le bac B20, le gaz traité débarrassé de la quasi totalité de l'eau et de 2-méthoxyéthanol sort par le conduit 26, traverse l'échangeur E11 dans lequel il se réchauffe par échange thermique avec le gaz circulant dans les conduits 25 et 27, ressort par le conduit 43, est recomprimé jusqu'à 8 MPa dans le compresseur K10 et évacué par le conduit 45 pour être expédié. Une fraction de ce gaz recomprimé est prélevée par le conduit 44, détendue à une pression voisine de celle du gaz traité et envoyée dans le bac B11. Au cours du contact à contre-courant dans le garnissage G11 avec liquide provenant du conduit 41, le gaz traité arrivant par le conduit 42, peu chargé en eau et en 2-méthoxyéthanol, se charge préférentiellement en eau, et ressort par le conduit 28 pour être mélangé au flux principal de gaz traité provenant du conduit 27. La phase liquide recueillie en fond du bac B11, plus concentrée en 2-méthoxyéthanol que le liquide circulant dans le conduit 41, est reprise par la pompe P11, traverse l'échangeur E14, ressort par le conduit 35 et est mélangée au flux de gaz circulant dans le conduit 34.

Dans certains cas le gaz à traiter peut contenir une proportion relativement importante de gaz acides tels que $CO_2$ et $H_2S$ et il peut être nécessaire de réduire cette teneur en gaz acides.

Dans un tel cas, il est avantageux d'inclure dans le procédé une étape additionnelle d'élimination des gaz acides, mettant en oeuvre le même solvant que celui qui est utilisé dans l'ensemble du procédé.

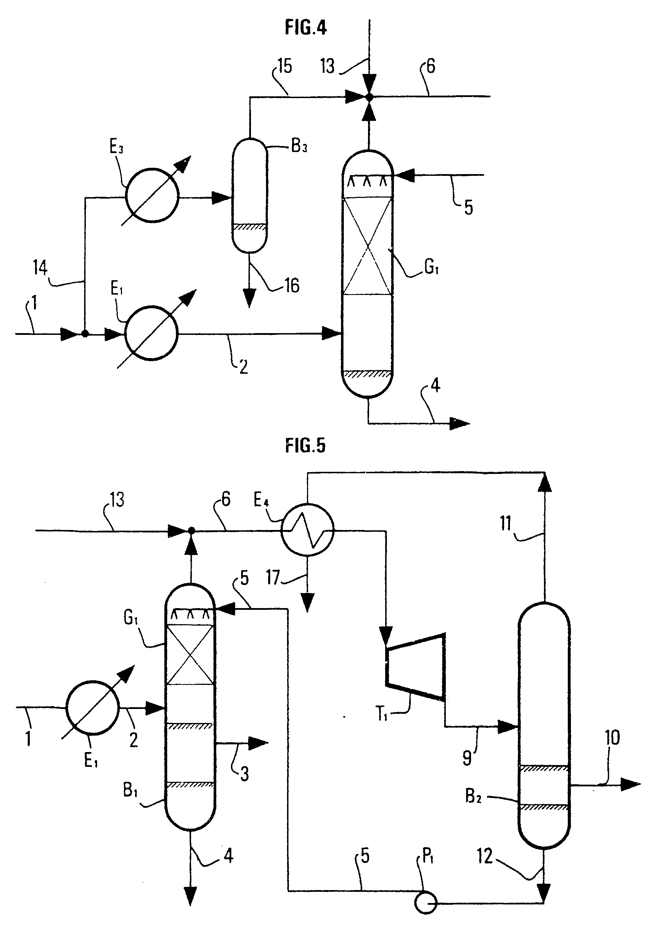

Une telle élimination des gaz acides peut être réalisée par exemple en opérant selon le schéma de la Figure 3.

Dans le cas de la version du procédé schématisée sur la Figure 1, cette étape peut intervenir par exemple à l'issue de l'étape (c). Le gaz évacué par le conduit 11 est contacté à contre-courant avec la phase solvant arrivant par le conduit 100 dans la zone de contact G100 formée par exemple par un garnissage.

Le gaz évacué par le conduit 101 est un gaz substantiellement libéré en gaz acides. La phase solvant chargées en gaz acides est régénérée par simple détente. Pour réduire les pertes en solvant, il est avantageux d'opérer des détentes successives. Sur le schéma de la Figure 3, la régénération est opérée en deux détentes successives. A l'issue de la première détente du gaz (conduit 102), opérée à travers la vanne de détente V100, la phase gazeuse et la phase liquide obtenues sont séparées dans le bac B100. La phase gazeuse est évacuée par le conduit 103.

La phase liquide est évacuée par le conduit 104 e t détendue à nouveau à travers la vanne V101. La phase gazeuse et la phase liquide obtenues sont séparées dans le bac B101. La phase gazeuse est évacuée par le conduit 105. La phase liquide est évacuée par le conduit 100 et recyclée vers la zone de contact G100 au moyen de la pompe P100.

Le taux de solvant est nécessairement plus élevé que celui qui est mis en oeuvre au cours de l'étape (a) du procédé. C'est ainsi que dans le cas où le solvant est du méthanol, le rapport molaire du débit de solvant sur le débit de gaz traité est typiquement de l'ordre de 0,5. Ce rapport est généralement compris entre 0,1 et 1.

Toutefois, dans ce cas également, on préfère éviter le recours à la distillation, la régénération du solvant au cours de cette étape pouvant être assurée par détente.

Au cours des détentes successives, les niveaux de pression sont régulièrement étagés à partir d'une pression haute voisine de la pression du gaz à traiter, la pression de détente la plus basse pouvant être voisine de la pression atmosphérique.

L'étape additionnelle introduite dans cette version du procédé est donc caractérisée en ce que le gaz issu des étapes précédentes est contacté avec un débit de solvant suffisant pour entraîner les gaz acides à éliminer, la phase solvant issue de cette opération de lavage étant régénérée par une ou plusieurs détentes successives et recyclée.

L'opération de lavage est réalisée avantageusement à une température voisine de la température du gaz arrivant dans la zone de contact. Ainsi lorsqu'elle est effectuée à l'issue de l'étape (c) du procédé, elle est avantageusement menée à une température voisine de la température obtenue à l'issue de l'étape (b). Ceci est avantageux, notamment lorsque le solvant utilisé est le méthanol, car cela permet d'utiliser le solvant dans des conditions de meilleure sélectivité et de perte en solvant réduite.

Au cours de détentes successives permettant la régénération du solvant, les gaz acides éliminés par les conduits 103 et 105 peuvent entraîner des vapeurs de solvant, ce qui conduit à une perte de solvant. Ces traces de solvant en phase gazeuse peuvent être récupérées par un lavage des gaz acides avec de l'eau de manière à absorber les traces de solvant. La solution aqueuse de solvant obtenue après ledit lavage peut être, comme dans le cas du lavage de la phase liquide de GPL, réinjectée dans la solution recyclée circulant dans le conduit 5 et être régénérée dans le contacteur G1, en évitant ainsi à nouveau le recours à la régénération par distillation.

En plaçant cette étape additionnelle à l'issue de l'étape (a) il est également possible d'opérer à une température plus élevée qui peut être plus appropriée avec un solvant dont la température d'ébullition est relativement élevée.

Dans le cas de la deuxième version du procédé décrite en référence avec le schéma de la Figure 2, l'étape additionnelle d'élimination des gaz acides est opérée préférentiellement à l'issue de l'étape (e) sur le gaz provenant de la séparation gaz-liquide opérée au cours de l'étape (f) avant réchauffage du gaz par échange interne, mais elle peut avoir lieu également à l'issue de l'étape (c) ou de l'étape (a).

Différents agencements sont donc possibles qui seront sélectionnés selon la composition du gaz à traiter et la nature des spécifications requises pour le gaz issu du traitement.

Le procédé selon l'invention s'applique à un gaz naturel. Ce gaz naturel peut être un gaz à condensat. Dans ces cas le but recherché est d'ajuster le point de rosée eau, le point de rosée hydrocarbure et éventuellement de produire simultanément une fraction liquide hydrocarbure.

Le gaz traité peut être également un gaz associé dont les conditions de traitement sont similaires à celles du gaz à condensat.

Le gaz traité peut être aussi un gaz dit "sec" car ne produisant pas de fraction liquide hydrocarbure dans les conditions normales de production auquel cas le but recherché est avant tout d'ajuster le point de rosée eau.

L'invention s'applique également à un gaz de raffinerie tel qu'un "fuel gas" issu des unités de conversion pouvant contenir également de l'hydrogène.

Dans ce cas le but recherché sera d'assurer la déshydratation et/ou la séparation d'une fraction liquide hydrocarbure.

Dans l'étape (a) du procédé selon l'invention, la pression du gaz traité peut être très variables et prendre des valeurs comprises entre 1 et 200 bars, la température étant choisie par exemple entre 0 et 100 °C, de préférence entre 10 et 50 °C.

## Revendications

1. Procédé de traitement d'un gaz contenant du méthane et de l'eau, dans le but de débarrasser au moins en partie le gaz de ladite eau, caractérisé en ce qu'il comprend les étapes suivantes :

   a) on contacte ledit gaz avec une phase liquide recyclée renfermant à la fois de l'eau et un solvant, ledit solvant étant un composé non-hydrocarbure autre que l'eau, miscible à l'eau, on maintient des conditions de contact permettant de vaporiser et transférer dans le gaz au moins 90 % de la quantité de solvant introduite, provenant de la phase liquide recyclée, on évacue la phase aqueuse liquide résultant dudit contact et renfermant moins de 10 % de la quantité de solvant introduite et on recueille un gaz renfermant, à l'état vaporisé, au moins 90 % de la quantité de solvant introduite ;

   b) on refroidit le gaz provenant de l'étape (a) en produisant la condensation d'une fraction liquide formée essentiellement de solvant et d'eau ;

   c) on sépare le gaz non-condensé de ladite fraction liquide et on recycle à l'étape (a) la fraction liquide renfermant du solvant et de l'eau pour y constituer la phase liquide recyclée.

2. Procédé selon la revendication 1, caractérisé en ce que le rapport en moles du débit de solvant sur le débit de gaz traité au cours de l'étape (a) est compris entre 1/1000 et 1/10.

3. Procédé selon l'une des revendications 1 à 2, caractérisé en ce que le solvant est un alcool.

4. Procédé selon l'une des revendications 1 à 3, caractérisé en ce que le solvant est le méthanol.

5. Procédé selon l'une des revendications 1 à 4, caractérisé en ce que le solvant est un solvant qui forme un azéotrope avec l'eau.

6. Procédé selon l'une des revendications 1 à 5, caractérisé en ce que le solvant est choisi parmi les solvants suivants : éthanol, 2-méthoxyéthanol, propanol, butanol, alcool propargylique, pyridine, pipéridine.

7. Procédé selon l'une des revendications 1 à 6, caractérisé en ce que le contact de l'étage (a) est un contact à contre-courant entre le gaz ascendant et la phase liquide recyclée descendante.

8. Procédé selon l'une des revendications 1 à 7, caractérisé en ce que, avant le refroidissement de l'étape (b), on ajoute au gaz provenant de l'étape (a) une fraction gazeuse provenant de l'étape (g) ci-après, et en ce que :

d) on mélange la fraction de gaz provenant de l'étape de séparation (c) avec une fraction contenant du solvant en phase liquide provenant de l'étape (g) ;

e) on refroidit le mélange liquide-gaz provenant de l'étape (d) en produisant une fraction liquide formée essentiellement de solvant et d'eau et une fraction gazeuse ;

f) après l'étape de réfrigération (e), on sépare les fractions obtenues et

g) on évacue une partie de la fraction gazeuse séparée à l'étape (f), comme gaz épuré, et on met en contact une autre partie de ladite fraction gazeuse séparée à l'étape (f) avec au moins une partie de la fraction liquide de solvant et d'eau, séparée à l'étape (f), à température plus élevée que celle de l'étape (f) en produisant une fraction liquide formée essentiellement de solvant et une fraction gazeuse, et on recycle ladite fraction liquide à l'étape (d) et ladite fraction gazeuse à l'étape (b).

9. Procédé selon l'une des revendications 1 à 8, appliqué à un gaz renfermant, en outre, au moins un hydrocarbure autre que le méthane, condensable dans les conditions de l'étape (b) en formant une fraction liquide d'hydrocarbure distincte de ladite fraction formée essentiellement de solvant et d'eau, caractérisé en ce que l'on sépare ladite fraction liquide d'hydrocarbure et en ce qu'on la décharge du procédé.

10. Procédé selon l'une des revendications 1 à 9, caractérisé en ce que le gaz traité passe par une étape de traitement complémentaire pour éliminer au moins en partie les gaz acides présents dans ledit gaz, le gaz traité étant mis en contact avec du solvant en quantité suffisante pour dissoudre les gaz acides à éliminer et la phase solvant issue de cette opération de contact étant détendue de manière à produire au moins une fraction gazeuse qui est éliminée et une phase liquide qui est recyclée à la zone du contact dudit traitement complémentaire.

11. Procédé selon l'une des revendications 1 à 10, caractérisé en ce que du solvant est ajouté au gaz provenant de l'étape (a), avant de procéder à l'étape (b), en quantité suffisante pour compenser les pertes et maintenir un fonctionnement stable.

12. Procédé selon l'une des revendications 1 à 11, caractérisé en ce que les conditions de contact sont choisies pour maintenir une concentration en solvant de 0,01 à 5 % en poids dans la phase aqueuse liquide évacuée à l'étape (a).

13. Procédé selon l'une des revendications 1 à 12, caractérisé en ce que, au cours de l'étape (a), une fraction seulement de la quantité totale de gaz à traiter est mise en contact avec la solution liquide recyclée, l'autre fraction du gaz étant mélangée au gaz recueilli après contact avec la solution liquide recyclée.

14. Procédé selon la revendication 13, caractérisée en ce que la fraction du gaz traité mise en contact avec la solution liquide recyclée est comprise entre 10 et 50 % de la quantité totale de gaz à traiter.

15. Procédé selon la revendication 9, caractérisé en ce que la phase liquide d'hydrocarbure formée au cours de l'étape (b) est mise en contact avec l'eau de façon à transférer dans l'eau une partie au moins de solvant dissout dans la phase liquide d'hydrocarbure, de manière à obtenir une phase liquide d'hydrocarbure au moins partiellement débarrassée de solvant et une phase liquide d'eau et de solvant qui peut être mélangée à la phase liquide recyclée à l'étape (a) de l'étape (c).

16. Procédé selon la revendication 10, caractérisé en ce que la fraction gazeuse éliminée obtenue après détente du solvant est mise en contact avec l'eau de façon à transférer dans l'eau une partie au moins du solvant contenu dans ladite fraction gazeuse, la solution d'eau et de solvant obtenue étant mélangée à la phase liquide recyclée à l'étape (a) de l'étape (c).

17. Procédé selon l'une des revendications 1 à 16, caractérisé en ce que la réfrigération nécessaire à l'étape (b) est produite au moins en partie par détente d'une partie au moins du gaz traité dans une turbine de détente et/ou une vanne de détente.

**Claims**

1. A process for the treatment of a methane and water-containing gas in order to remove at least a part of said water from the gas, characterized by the following steps of :

a) contacting said gas with a recycled liquid phase containing both water and solvent, said solvent being a non hydrocarbon compound other than water, miscible with water, maintaining contact conditions adapted to vaporize and transfer to the gas at least 90% of the introduced solvent amount, originating from the recycled liquid phase, discharging the liquid aqueous phase resulting from said contact and containing at least 10% of the introduced solvent amount and recovering a gas containing in vaporized state, at least 90% of the introduced solvent amount;

b) cooling the gas obtained in step (a), thereby producing the condensation of a liquid fraction essentially formed of solvent and water;

c) separating the uncondensed gas from said liquid fraction and recycling to step (a) the liquid fraction containing solvent and water,to form the recycled liquid phase.

2. A process according to claim 1, characterized in that the ratio by moles of the solvent flow rate to the flow rate of treated gas during step (a) ranges from 1/1000 to 1/10.

3. A process according to one of claims 1 and 2, characterized in that the solvent is an alcohol.

4. A process according to one of claims 1 to 3, characterized in that the solvent is methanol.

5. A process according to one of claims 1 to 4, characterized in that the solvent is a solvent forming an azeotrope with water.

6. A process according to one of claims 1 to 5, characterized in that the solvent is selected from the following solvents: ethanol, 2-methoxy-ethanol, propanol, butanol, propargyl alcohol, pyridine, piperidine.

7. A process according to one of claims 1 to 6, characterized in that the contact of step (a) is a counter-current contact between the gas up-flow and the recycled liquid phase down-flow.

8. A process according to one of claims 1 to 7, characterized in that, before cooling in step (b), a gas fraction originating from subsequent step (g) is added to the gas obtained in step (a), and in that:

d) the gas fraction originating from the separation step (c) is admixed with a fraction containing solvent in liquid phase originating from step (g);

e) the liquid-gas mixture originating from step (d) is cooled so as to produce a liquid fraction essentially formed of solvent and water and a gas fraction;

f) the obtained fractions are separated after the cooling step (e), and

g) a part of the gas fraction separated in step (f)is discharged as purified gas and another part of said gas fraction separated in step(f) is contacted with at least a portion of the solvent and water liquid fraction separated in step (f), at a temperature higher than that of step (f), thus producing a liquid fraction essentially formed of solvent and a gas fraction, and recycling said liquid fraction to step (d) and said gas fraction to step (b).

9. A process according to one of claims 1 to 8, applied to a gas further containing at least one hydrocarbon other than methane, condensable in the conditions of step (b) by forming a hydrocarbon liquid fraction separate from said fraction consisting essentially of solvent and water, characterized in that said hydrocarbon liquid fraction is separated and discharged from the process.

10. A process according to one of claims 1 to 9, characterized in that the treated gas is subjected to an additional treatment step for removing at least a portion of the acid gases present in said gas, the treated gas being contacted with solvent in sufficient amount to dissolve the acid gases to be removed and the solvent phase obtained from this contact operation being expanded so as to produce at least one gas fraction which is removed and a liquid phase which is recycled to the contact zone of said additional treatment.

11. A process according to one of claims 1 to 10, characterized in that the solvent is added to the gas obtained in step (a),before proceeding to step (b),in sufficient amount to compensate for the losses and to maintain a stable operation.

12. A process according to one of claims 1 to 11, characterized in that the contact conditions are so selected as to maintain a solvent concentration from 0.01 to 5% by weight in the aqueous liquid phase discharged from step (a).

13. A process according to one of claims 1 to 12, characterized in that, during step (a), only a fraction of the total amount of gas to be treated is contacted with the recycled liquid solution, the other gas fraction being admixed with the gas recovered after contact with the recycled liquid solution.

14. A process according to claim 13, characterized in that the portion of treated gas contacted with the recycled liquid solution amounts to 10-50% of the total amount of gas to be treated.

15. A process according to claim 9, charactrized in that the hydrocarbon liquid phase formed during step (b) is contacted with water so as to transfer to water at least a part of the solvent dissolved in the hydrocarbon liquid phase; so as to obtain a hydrocarbon liquid phase at least partially free of solvent and a water and solvent liquid phase which may be admixed to the liquid phase recycled from step (c) to step (a).

16. A process according to claim 10, characterized in that the discharged gas fraction obtained after expansion of the solvent is contacted with water so as to transfer to water at least a portion of the solvent contained in said gas fraction, the obtained water and solvent solution being admixed with the liquid phase recycled from step (c) to step (a).

17. A process according to one of claims 1 to 16, characterized in that the required cooling of step (b)is obtained at least partly by expanding at least a portion of the treated gas through an expansion turbine and/or an expansion walve.

## Ansprüche

1. Verfahren zum Behandeln eines Methan und Wasser enthaltenden Gases mit dem Ziel, wenigstens zum Teil das Gas von diesem Wasser zu befreien, dadurch gekennzeichnet, daß es die folgenden Stufen umfaßt:

a) man kontaktiert dieses Gas mit einer flüssigen rezyklisierten Phase, die gleichzeitig Wasser und ein Lösungsmittel enthält, wobei dieses Lösungsmittel eine Nicht-Kohlenwasserstoffverbindung außer Wasser ist, die mit Wasser mischbar ist, man hält Kontaktbedingungen aufrecht, die es ermöglichen, wenigstens 90% der eingeführten aus der flüssigen rezyklisierten Phase stammenden Lösungsmittelmenge zu verdampfen und in das Gas zu überführen, man evakuiert die wässrige flüssige aus diesem Kontakt stammende Phase, welche wenigstens 10% der eingeführten Lösungsmittelmenge enthält und sammelt ein Gas, das im verdampften Zustand wenigstens 90% der eingeführten Lösungsmittelmenge enthält;

b) man kühlt das aus der Stufe (a) kommende Gas, indem man die Kondensation einer flüssigen Fraktion, die im wesentlichen aus Lösungsmittel und Wasser gebildet ist, hervorruft;

c) und man trennt das nicht-kondensierte Gas von dieser flüssigen Fraktion und rezyklisiert in die Stufe (a) die flüssige, Lösungsmittel und Wasser enthaltende Fraktion, um hierin die flüssige rezyklisierte Phase zu bilden.

2. Verfahren nach Anspruch 1, dadurch gekennzeichnet, daß das Molverhältnis des Lösungsmitteldurchsatzes zum Durchsatz des während der Stufe (a) behandelten Gases zwischen 1/1000 und 1/10 beträgt.

3. Verfahren nach einem der Ansprüche 1 bis 2, dadurch gekennzeichnet, daß das Lösungsmittel ein Alkohol ist.

4. Verfahren nach einem der Ansprüche 1 bis 3, dadurch gekennzeichnet, daß das Lösungsmittel Methanol ist.

5. Verfahren nach einem der Ansprüche 1 bis 4, dadurch gekennzeichnet, daß das Lösungsmittel ein ein Azeotrop mit dem Wasser bildendes Lösungsmittel ist.

6. Verfahren nach einem der Ansprüche 1 bis 5, dadurch gekennzeichnet, daß das Lösungsmittel gewählt ist aus den folgenden Lösungsmitteln: Ethanol, 2-Methoxyethanol, Propanol, Butanol, Propargylalkohol, Pyridin, Piperidin.

7. Verfahren nach einem der Ansprüche 1 bis 6, dadurch gekennzeichnet, daß der Kontakt der Stufe (a) ein Gegenstromkontakt zwischen dem aufsteigenden Gas und der rezyklisierten absteigenden flüssigen Phase ist.

8. Verfahren nach einem der Ansprüche 1 bis 7, dadurch gekennzeichnet, daß man vor dem Kühlen der Stufe (b) dem aus der Stufe (a) stammenden Gas eine gasförmige aus der unten bezeichneten Stufe (g) stammende Fraktion zusetzt und daß

d) man die aus der Trennstufe (c) stammende Gasfraktion mit einer Fraktion mischt, welche Lösungsmittel in flüssiger Phase, das der Stufe (g) entstammt, enthält;

e) man kühlt das aus der Stufe (d) stammende Flüssigkeits-Gasgemisch, indem man eine flüssige Fraktion erzeugt, die im wesentlichen aus Lösungsmittel und Wasser und einer gasförmigen Fraktion gebildet ist;

f) nach der Kühlstufe (e) trennt man die erhaltenen Fraktionen und

g) man evakuiert einen Teil der gasförmigen in Stufe (f) abgetrennten Fraktion als gereinigtes Gas und kontaktiert einen anderen Teil dieser gasförmigen in Stufe (f) abgetrennten Fraktion mit wenigstens einem Teil der flüssigen Fraktion aus Lösungsmittel und Wasser, die in Stufe (f) abgetrennt wurde, bei höherer Temperatur als der der Stufe (f), indem man eine flüssige Fraktion erzeugt, die im wesentlichen aus Lösungsmittel und einer gasförmigen Fraktion gebildet ist und man rezyklisiert diese flüssige Fraktion in die Stufe (d) und diese gasförmige Fraktion in die Stufe (b).

9. Verfahren nach einem der Ansprüche 1 bis 8, in Anwendung auf ein Gas, das unter anderem wenigstens einen Kohlenwasserstoff außer dem Methan enthält, und welches unter den Bedingungen der Stufe (b) kondensierbar ist, indem es eine flüssige Kohlenwasserstoff-Fraktion bildet, die sich von dieser im wesentlichen aus Lösungsmittel und Wasser bestehenden Fraktion unterscheidet, dadurch gekennzeichnet, daß man diese flüssige Kohlenwasserstoff-Fraktion abtrennt und sie aus dem Prozeß austrägt.

10. Verfahren nach einem der Ansprüche 1 bis 9, dadurch gekennzeichnet, daß das behandelte Gas eine komplementäre Behandlungsstufe passiert, um wenigstens zum Teil die sauren in diesem Gas enthaltenen Gase zu eliminieren, wobei das behandelte Gas mit Lösungsmittel in ausreichender Menge kontaktiert wird, um die sauren zu eliminierenden Gase aufzulösen und daß die aus diesem Kontaktvorgang stammende Lösungsmittelphase derart entspannt wird, daß wenigstens eine gasförmige Fraktion, die eliminiert wird, sowie eine flüssige Fraktion erzeugt wird, die in die Kontaktzone dieser komplementären Behandlung rezyklisiert wird.

11. Verfahren nach einem der Ansprüche 1 bis 10, dadurch gekennzeichnet, daß Lösungsmittel dem aus der Stufe (a) stammenden Gas, bevor zur Stufe (b) übergegangen wird, in ausreichender Menge zugesetzt wird, um die Verluste zu kompensieren und einen stabilen Betrieb aufrecht zu erhalten.

12. Verfahren nach einem der Ansprüche 1 bis 11, dadurch gekennzeichnet, daß die Kontaktbedingungen derart gewählt sind, daß eine Lösungsmittelkonzentration von 0,01 bis 5 Gew.-% in der flüssigen wässrigen in der Stufe (a) evakuierten Phase aufrecht erhalten wird.

13. Verfahren nach einem der Ansprüche 1 bis 12, dadurch gekennzeichnet, daß während der Stufe (a) nur eine Fraktion der Gesamtmenge des zu behandelnden Gases mit der rezyklisierten flüssigen Lösung kontaktiert wird, wobei die andere Fraktion des Gases mit dem Gas vermischt wird, das nach Kontakt mit der flüssigen rezyklisierten Lösung gesammelt wird.

14. Verfahren nach Anspruch 13, dadurch gekennzeichnet, daß die Fraktion des behandelten mit der flüssigen rezyklisierten Lösung kontaktierten Gases zwischen 10 und 50 % der Gesamtmenge des zu behandelten Gases umfaßt.

15. Verfahren nach Anspruch 9, dadurch gekennzeichnet, daß die während der Stufe (b) gebildete flüssige Kohlenwasserstoffphase mit Wasser derart kontaktiert wird, daß in das Wasser wenigstens ein Teil des Lösungsmittels überführt wird, welches in der flüssigen Kohlenwasserstoffphase gelöst ist, derart, daß eine flüssige Kohlenwasserstoffphase erhalten wird, die wenigstens teilweise vom Lösungsmittel befreit ist und eine flüssige Phase aus Wasser und Lösungsmittel erhalten wird, die mit der flüssigen in die Stufe (a) aus der Stufe (c) rezyklisierten Phase gemischt werden kann.

16. Verfahren nach Anspruch 10, dadurch gekennzeichnet, daß die nach Entspannen des Lösungsmittels erhaltene eliminierte gasförmige Fraktion mit Wasser derart kontaktiert wird, daß in das Wasser wenigstens ein Teil des in dieser gasförmigen Fraktion enthaltenen Lösungsmittels überführt wird, wobei die erhaltene Lösung aus Wasser und Lösungsmittel mit der flüssigen in die Stufe (a) aus der Stufe (c) rezyklisierten Phase vermischt wird.

17. Verfahren nach einem der Ansprüche 1 bis 16, dadurch gekennzeichnet, daß die in der Stufe (b) notwendige Kühlung wenigstens zum Teil durch Entspannen wenigstens eines Teils des behandelten Gases in einer Entspannungsturbine und/oder einem Entspannungsventil erzeugt wird.

## FIG.1

## FIG.3

FIG.2

## FIG.4

## FIG.5